Europäisches Patentamt

European Patent Office

Office européen des brevets

(11) Veröffentlichungsnummer: **0 242 415 B1**

# (12) EUROPÄISCHE PATENTSCHRIFT

(51) Int. Cl.⁴: **A61F 13/00**

(45) Veröffentlichungstag der Patentschrift:
25.01.89

(21) Anmeldenummer: 86105530.9

(22) Anmeldetag: **22.04.86**

(54) **Textiles Flächengebilde zur Wundversorgung.**

(43) Veröffentlichungstag der Anmeldung:
**28.10.87 Patentblatt 87/44**

(45) Bekanntmachung des Hinweises auf die Patenterteilung:
**25.01.89 Patentblatt 89/4**

(84) Benannte Vertragsstaaten:
**AT BE CH DE FR GB IT LI LU NL SE**

(56) Entgegenhaltungen:
**EP-A- 0 099 758**
**FR-A- 2 119 721**
**FR-A- 2 281 099**
**FR-A- 2 525 245**

(73) Patentinhaber: **Karl Otto Braun KG, Lauterthalstrasse, D-6759 Wolfstein(DE)**

(72) Erfinder: **Langen, Günter, Dr., Am Herrenacker 7, D-6759 Wolfstein(DE)**
Erfinder: **Jung, Harald, Im Flürchen 9, D-6751 Kreimbach-Kaulbach 1(DE)**

(74) Vertreter: **Richter, Joachim, Dipl.-Ing., Patentanwälte Richter u.Werdermann Neuer Wall 10, D-2000 Hamburg 36(DE)**

## Beschreibung

Die Erfindung betrifft ein textiles Flächengebilde zur Wundversorgung.

Für den Bereich der textilen Wundabdeckungen eignen sich generell textile Flächengebilde auf der Basis von Geweben, Gewirken, Gestricken, Non wovens sowie kombinierte Konstruktionen.

Ein Wundverband soll die Wunde vor Verunreinigungen schützen und den Heilverlauf fördern, wobei ein idealer Wundverbandstoff folgende Eigenschaften besitzen soll: Der Verbandstoff

– muß hohe Saugfähigkeit und Porosität aufweisen, da Flüssigkeitsansammlungen ideale Voraussetzungen für eine Keimbesiedlung und damit für eine Wundinfektion schaffen,
– darf keinen Wärmestau verursachen, d.h. die Temperatur im Bereich der Wunde soll durch die Auflage keine Erhöhung erfahren,
– muß Mindestforderungen bezüglich der Reißfestigkeit erfüllen, insbesondere Naßreißfestigkeit,
– muß leicht und einfach anwendbar sein und sich nach den bekannten Methoden sterilisieren lassen,
– soll sich leicht und faltenlos der Wunde anschmiegen und keine Schrumpfungstendenz zeigen,
– muß physiologisch einwandfrei, also reizlos sein,
– muß luftdurchlässig sein, wobei vor allem die Porengröße des Flächengebildes in einer bestimmten Beziehung zur Fadenstärke stehen muß. Bei zu kleiner Porengröße kommt es zu Sekretstauung, Verklebung und Luftabschluß.

Zur Wundabdeckung ist es bekannt, Verbandmull aus einem weitmaschigen Baumwoll-, Zellwoll- oder Mischgewebe zu verwenden, wobei der Verbandmull zur Wundabdeckung in mehrfachen Lagen zu Kompressen verschiedener Größe und Dicke gelegt wird. Verbandmull aus reiner Baumwolle hat sich aufgrund der im folgenden aufgeführten Eigenschaften jahrzehntelang als Wundbehandlungsmaterial bewährt und ist nach wie vor wegen seiner hohen Wirtschaftlichkeit das am meisten verwendete Verbandmaterial bei der Wundversorgung:

– Hohe Saugfähigkeit, großes Wasserhaltevermögen,
– hohe Stabilität in trockenem und nassem Zustand,
– hohe Bauschelastizität, gute Polsterwirkung,
– hohe Luft- und Wasserdampfdurchlässigkeit,
– gutes plastisches Anlegevermögen,
– vielseitige Verwendungsformen, dabei sehr preisgünstig,
– chemische Reinheit.

Die Wundabdeckung mit reinem Verbandmull erfüllt jedoch nicht alle Anforderungen, die an Wundauflagen gestellt werden. Durch Verklebungen und Verkrustungen kann es zu Sekretstauungen kommen und bei der Abnahme eines verklebten Verbandes werden die frischen Granulationen und Epithelsäume abgerissen, so daß erneut Blutungen, Verletzungen und Schmerzen entstehen. Dadurch, daß Verbandmull mit der Wunde verklebt, wird bei einem Verbandwechsel die Wunde wieder aufgerissen und somit die Wundruhe gestört, d.h. der Heilungsprozeß wird unterbrochen. Verbandmull wird in der Praxis als sogenannter Stückmull in 40 mtr. Länge und in diversen Breiten angeboten und dient vorwiegend zur Selbstanfertigung von Kompressen, Tupfern, Bauchtüchern u.dgl. Eine bereits vorkonfektionierte Form ist der Zick-Zack-Mull oder abgepaßte und/oder mechanisch gestauchte, gefaltete Rollenware in sogenannten Dispensern. Bei den gebräuchlichen Mullkompressen, die z.B. 4-, 6-, 8-, 12-, 16-lagig gefaltet sind, werden die Schnittkanten beim Faltungsprozeß so eingelegt, daß sie im Innern der Kompresse oder des Tupfers liegen, um ein Austreten von Fasertrümmern zu vermeiden.

Neben Verbandmull sind wundfreundliche Auflagen bekannt, so z.B. Auflagen mit einem Bewegungseffekt. Durch Verwendung unterschiedlich stark gedrillter und verschieden dicker Fäden wird aus reiner Zellwolle ein Flächengebilde durch Weben oder Wirken hergestellt, das sich bei Wasser- oder Sekretzusatz krümmt und bewegt. Durch diesen Bewegungseffekt wird das Gewebe tunnelartig bei Einwirkung von Flüssigkeit von der Wunde abgehoben und eine Verklebung verhindert. Die Verklebung des Textilfadens mit der Wunde wird hier durch Bewegung, also rein mechanisch, verhindert.

Nach der DE-A 2 261 889 ist eine Kompresse, insbesondere für ärztlichen, chirurgischen und anderweitigen Gebrauch bekannt, bei der von einem vermaschten Streifen ausgegangen wird, der derart auf einem Maschenwebstuhl ausgeführt ist, daß der genannte Streifen Ränder und eine luftdurchlässige und offene Textur mit kleinen, sich längs erstreckenden Ketten darbietet, die durch Querfäden miteinander verbunden sind, wobei der Streifen oder die übereinandergelegten Streifen mit Rändern auf die passende Länge geschnitten und auf solche Weise zurückgefaltet sind, daß die Kompresse mit ihren Abmessungen gebildet wird. Eine derart ausgebildete Kompresse soll die Gefahr des Zerfaserns beseitigen. Bei dieser Kompresse handelt es sich um ein mullähnliches Gewirke, bei dem das Austreten von Fasertrümmern durch sogenannte blockierte Maschen verhindert werden soll. Die technisch-physikalischen und chemischen Eigenschaften sind weitgehend identisch mit den Merkmalen des Verbandmulls.

Ferner ist durch die DE-A 1 492 434 ein Wundverbandstoff, bestehend aus einem textilen Gewirk, bei dem ein starker Faden von einem dünnen, in Maschenlegung geführten Faden eingebunden ist, bekannt, bei dem der starke Faden hochgedreht ist, gradlinig oder in leicht versetztem Stehschuß geführt und von mindestens einem minimal gedrehten Faden eingebunden ist. Dadurch, daß dieser Wundverbandstoff unterschiedliche Garndimensionen und Garndrehungen aufweist, verkürzen sich die Fäden bei einem Feuchtigkeitszutritt, wodurch ein Abheben des Wundverbandstoffes von der Wundoberfläche erreicht wird. Dadurch tritt ein nichtverklebender Effekt ein. Aufgrund der relativ dichten Fadenkonstruktion, läßt sich dieser Wundverbandstoff nur begrenzt als Kompresse, und zwar wegen mangelnder Plastizität, einsetzen.

Die DE-A 3 213 673 beschreibt einen Wundverbandstoff, insbesondere für Wundschnellverbände, mit der speziellen Zielsetzung, den Verbandwechsel schonend zu gestalten. Hierbei handelt es sich um einen Wundverbandstoff, der zu der Gruppe der atraumatischen Verbandstoffe gehört. Die mit diesem Wundverbandstoff erreichte spezielle Eigenschaft resultiert aus einer Kombination zwischen wundseitig angeordneten, hydrophoben Polyester-Fasern und hochsaugfähigen Zellwollgarnen in einer textilen Verbund-Konstruktion.

Eine weitere Gruppe von textilen Wundauflagen betrifft die sogenannten Non wovens. Non wovens sind textile Flächengebilde in Form eines Faserverbandes, wobei die Einzelfasern mechanisch, thermisch oder chemisch miteinander verbunden sind. In den meisten Fällen werden für dieses textile Flächengebilde Zellwollfasern verwendet. Aufgrund des verwendeten Materials und des Fasernverbundes ist die Festigkeit derartiger textiler Flächengebilde äußerst gering. Insbesondere die Naßreißfestigkeit liegt erheblich unter der von Geweben und Gewirken. Auch die Bauschelastizität ist gering. Das Problem der abstehenden Fasern kann nur durch eine zusätzliche Behandlung mit Kunstharzdispersionen bzw. durch Aufkaschieren von perforierten Folien gelöst werden.

Darüber hinaus sind eine Reihe von kombinierten Wundverbandstoffen auf der Basis von Verbandmull bekannt, wie z.B. Watte-Mull oder Zellstoff-Mull-Kompressen. Das Wasserhaltevermögen dieser Kompressen ist durch den als innenliegender Saugkörper dienenden Zellstoff- oder Watte-Anteil erhöht, während dagegen die Mullschicht mit der Wunde in Berührung steht und ein Verkleben der Mullschicht mit der Wunde nicht ausschließbar ist.

Die Erfindung löst die Aufgabe, ein offenporiges, luftdurchlässiges, gewirktes, nicht mit der Wunde verklebendes, textiles Flächengebilde mit verbandmullähnlichen jedoch verbesserten Eigenschaften zu schaffen, das optimale Wundruhe gewährleistet und das gefaltet eine Wundkompresse mit einer auf der Wunde aufliegenden hydrophoben Abdeckschicht und mit über dieser liegenden hydrophilen Saugschichten ergibt, die gegenüber Verbandmull außerdem noch eine höhere Mindestreißfestigkeit aufweist.

Zur Lösung dieser Aufgabe wird ein textiles Flächengebilde zur Wundversorgung vorgeschlagen, das erfindungsgemäß in der Weise ausgebildet ist, daß in einer breit gewirkten oder geraschelten Bahn aus saugfähigen, hydrophilen cellulosischen Fasergarnen, wie z.B. Baumwolle, Zellwolle, Baumwolle/Zellwolle od.dgl., mindestens ein streifenförmiger Bahnabschnitt aus hydrophoben Chemiefasergarnen aus synthetischen Polymeren mit einer hohen Oberflächenspannung, wie z.B. aus Polyamid, Polyester, Polyäthylen, Polypropylen, Polyacrylnitril, Polytetrafluoräthylen od.dgl., durch Vermaschung in einer Ebene eingearbeitet ist, wobei der streifenförmige Bahnabschnitt als Mittelstreifen oder als Randstreifen in der Bahn des textilen Flächengebildes ausgebildet ist, das zu einer Kompresse derart faltbar ist, daß der Bahnabschnitt aus den hydrophoben Chemiefasern als hydrophobe Abdeckschicht die Wundauflage und die hinter dem Bahnabschnitt liegenden Lagen aus den cellulosischen Fasergarnen die Saugschicht oder die Saugschichten bildend sind.

Bei einem derart ausgebildeten textilen Flächengebilde handelt es sich um einen Wundverbandstoff ähnlich dem bekannten Verbandmull nach DIN 61630, jedoch mit dem weiteren und zusätzlichen Vorteil gegenüber den Vorteilen des Verbandmulls des Nichtverklebens auf der Wunde. Das textile Flächengebilde zur Wundversorgung ist gegenüber dem bekannten Verbandmull dadurch gekennzeichnet, daß in einer breit gewirkten oder geraschelten Bahn aus saugfähigen, cellulosischen Fasergarnen, wie z.B. Baumwolle oder Zellwolle oder Baumwolle/Zellwolle, ein streifenförmiger Bahnabschnitt aus Chemiefasergarnen, bestehend aus synthetischen Polymeren mit einer hohen Oberflächenspannung eingearbeitet ist. Als Chemiefasergarne finden dabei vorzugsweise polyfile Filamentgarne Verwendung; es können aber auch monofile Filamentgarne sowie Stapelfasergarne eingesetzt werden. Der streifenförmige Abschnitt kann dabei beispielsweise als Mittelstreifen oder auch als Randstreifen der Breitgewirkebahn ausgebildet sein, wobei entsprechend den bekannten Kompressen das Gewirke so gefaltet wird, daß der hydrophobe Chemiefaserteil wundseitig zu liegen kommt. Die dahinterliegenden Lagen aus cellulosischen Fasern wirken als Saugschicht. Die Maschengröße ist dabei so gewählt, daß einerseits keinerlei Verwachsungen mit dem sich bildenden Fibringerüst während der ersten Phase der Wundheilung auftreten können, andererseits aber auch ein ungehinderter Sekretabfluß durch die hydrophobe Schicht hindurch in die hydrophile Saugschicht entsprechend der bekannten Dochtwirkung erfolgen kann.

Es besteht auch die Möglichkeit, die nichtverklebende Wundabdeckschicht beidseitig anzubringen, indem durch entsprechendes Falten oder durch das Einarbeiten eines zweiten hydrophoben Streifens in die Kompresse die Saugschicht vollständig in das Innere der Kompresse verlegt wird.

Durch diese Konstruktion eines textilen Flächengebildes ist die hydrophobe nichtverklebende Wundabdeckschicht fest mit der Saugschicht derart verbunden, daß eine Trennung, wie z.B. bei folienbeschichteten Vliesstoffen, nicht möglich ist.

Überraschenderweise hat es sich gezeigt, daß durch den Chemiefaseranteil die Wundkompresse aus dem textilen Flächengebilde eine deutlich höhere Mindestreißfestigkeit aufweist, als diese für Verbandmull gefordert wird, was aus der nachstehenden Tabelle ersichtlich ist.

TABELLE 1: Vergleichende Übersicht der mechanisch-technologischen Daten verschiedener Wundkompressen

| | Mullkompresse nach DIN 61 630 (Bw.) 17-fädig, 8-fach | Vliesstoff-Kompresse | Erfindungsgemäße Kompresse, 6-lagig |
|---|---|---|---|
| Wasseraufnahmevermögen nach DIN 53 923 ($g/m^2$) | 1084 | 1057 | 1100 |
| Saugfähigkeit, Absinkdauer (sec.) | 1 | 2 | 1 |
| Reißkraft nach DIN 53 857 (N/cm) | > 80 | 33 | > 160 |
| Luftdurchlässigkeit nach DIN 53 887 ($l/m^2$ sec.) | 3500 | 1500 | 3500 |

Da die hydrophoben, synthetischen Polymeren eine sehr geringe Wasseraufnahmefähigkeit besitzen und kaum zur Quellung neigen, wird auch die geforderte Wundruhe optimal erreicht. In das textile Flächengebilde können auch die bei Mullkompressen üblichen Röntgenkontrastfäden, z.B. bariumsulfathaltige Zellwollfäden bzw. Fäden aus synthetischen Polymeren fest eingearbeitet werden, wobei ein Heraustreten des Röntgenkontrastfadens vollständig unterbunden ist.

Um dem Arzt und Patienten die Plazierung einer einlagig, nichtverklebenden Kompresse zu erleichtern, kann der hydrophobe Teil der Kompresse partiell oder vollflächig mit einem indifferenten Farbstoff gekennzeichnet sein. In gleicher Weise kann auch die Saugschicht eingefärbt oder mit pharmazeutischen Wirkstoffen, wie z.B. antibakteriellen Substanzen, imprägniert sein.

Weitere vorteilhafte Ausgestaltungen der Erfindung gehen aus den Unteransprüchen hervor.

Im folgenden wird der Gegenstand der Erfindung in den Zeichnungen erläutert. Es zeigt

Fig. 1 in einer Ansicht von oben ein textiles Flächengebilde mit einem die mit der Wunde nichtverklebenden Wundabdeckschicht bildenden Mittelstreifen und zu dessen beiden Seiten ausgebildeten Saugschichten,

Fig. 2 verschiedene Möglichkeiten eines Faltungsablaufs des textilen Flächengebildes zu einer Wundkompresse und

Fig. 3 schematisch die Verwendung einer aus dem textilen Flächengebilde gefalteten Wundkompresse bei der Versorgung einer durch eine Schnittwunde gekennzeichneten, verletzten Hautpartie.

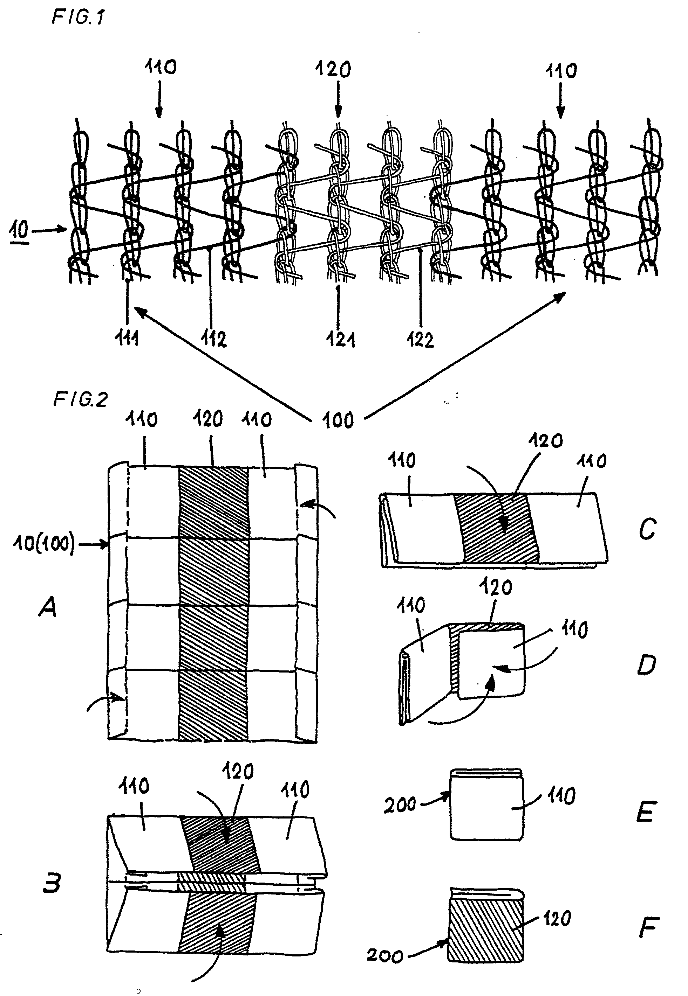

Der in Fig. 1 dargestellte Abschnitt einer mit 100 bezeichneten Breitgewirkebahn besteht aus einem breit gewirkten oder geraschelten textilen Flächengebilde aus saugfähigen, d.h. hydrophilen, cellulosischen Fasergarnen 111, 112. In dem textilen Flächengebilde bzw. in der Bahn 100 ist mindestens ein streifenförmiger Bahnabschnitt als Mittelstreifen 120 aus hydrophoben Chemiefasergarnen aus synthetischen Polymeren mit einer hohen Oberflächenspannung durch Vermaschung in einer Ebene eingearbeitet, wobei als synthetische Polymere Polyamid, Polyester, Polyäthylen, Polypropylen, Polyacrylnitril, Polytetrafluoräthylen u.dgl. Verwendung finden. Der streifenförmige Bahnabschnitt kann sowohl als Mittelstreifen als auch als Randstreifen in der Breitgewirkebahn 100 des textilen Flächengebildes 10 ausgebildet sein. Hiernach besteht nach Fig. 1 die Breitgewirkebahn 100 aus einem Mittelstreifen 120 aus hydrophoben Fäden und den links und rechts benachbarten Saugschichten 110 aus hydrophilen, cellulosischen Fäden. Bei 111 ist ein Maschenstab in Fransenbindung dargestellt, der aus cellulosischen Fäden gebildet ist. Die entsprechende Schußlegung aus cellulosischen Fäden ist bei 112 angedeutet. Der Maschenstab 121 ist z.B. in Fransenbindung aus hydrophoben Chemiefasergarnen geführt, des gleichen die in diesem Bereich eingearbeitete, mit 122 bezeichnete Schußlegung.

Fig. 2 zeigt beispielsweise einen möglichen Faltungsablauf A, B, C, D, E, F einer Breitgewirkebahn aus dem textilen Flächengebilde mit einer als Mittelstreifen 120 ausgebildeten, hydrophoben Wundabdeckschicht, die als schraffierte Fläche angedeutet ist. F zeigt die der Wunde zugekehrte Seite der fertig gefalteten Wundkompresse 200, die aus hydrophoben Chemiefasergarnen besteht. E zeigt die der Wunde abgewandte Saugschicht, und zwar bestehend aus hydrophilen, saugfähigen cellulosischen Fäden.

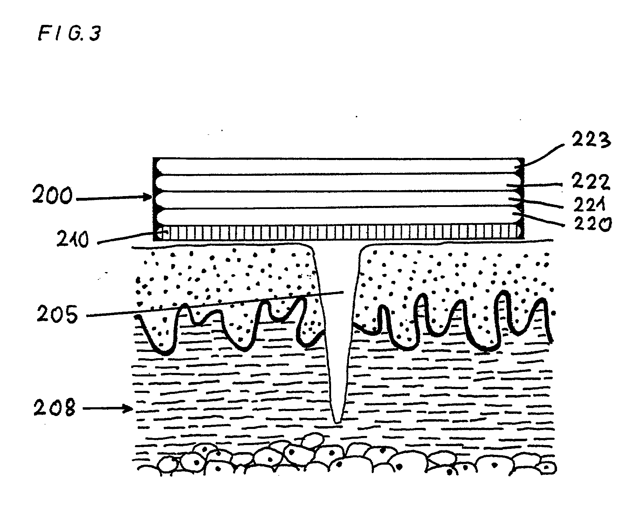

Die Verwendung einer aus dem textilen Flächengebilde 10 bzw. der Breitgewirkebahn 100 gebildeten Wundkompresse 200 bei der Versorgung einer durch eine Schnittwunde 205 gekennzeichneten, verletzten Hautpartie 208 ist in Fig. 3 schematisch dargestellt. Die hydrophobe Abdeckschicht 210 liegt dabei direkt auf der Wunde auf, während die hydrophilen, saugfähigen Schichten 220, 221, 222, 223 in den darüberliegenden Faltlagen angeordnet sind.

Als Rohstoffe für die saugfähige Schicht finden Baumwolle, Zellwolle, Leinen oder andere saugfähige Materialien als Stapelfaser- oder Filamentgarne Verwendung. Das textile Flächengebilde ist unter Verwendung bekannter Grundbindungsarten, wie z.B. Fransenbindung, Trikotbindung u.dgl. hergestellt. Die Dimensionen der verwendeten Garne liegen dabei zwischen 10 tex und 40 tex, vorzugsweise zwischen 14 tex und 20 tex. Als Chemiefasern finden monofile und polyfile Filamentgarne sowie Stapelfasergarne Verwendung. Die in Schußlegung geführten Fäden sind aus den entsprechenden Materialien aufgebaut. Die Chemiefasergarne aus synthetischen Polymeren können mit Silber, Aluminium oberflächlich beschichtet sein, wobei auch aufgedampfte Schichten aus Silber, Aluminium oder anderen geeigneten Werkstoffen oder Verbindungen Verwendung finden können.

Das die Wundkompresse bildende textile Flächengebilde ist antibakteriell oder medikamentös imprägniert. Als Imprägnierungsmittel können quarternäre Ammoniumverbindungen, z.B. N-Cethylpyridiniumchlorid, Benzalkoniumchlorid u.dgl. sowie Stoffklassen mit desinfizierenden Eigenschaften Verwendung finden. Auch kann Vaseline aufgebracht sein, der Medikamente, wie z.B. Perubalsam, Sulfonamide, Antibiotika, Anästhetika u.dgl. zugesetzt sind.

Zur Kennzeichnung der Wundauflagenseite können als Farbstoffe Pigmentfarbstoffe, die der Spinnmasse bereits beim Spinnprozeß hinzugefügt werden können, geeignete Dispersions- oder Säurefarbstoffe od.dgl. eingesetzt werden.

## Patentansprüche

1. Textiles Flächengebilde (10) zur Wundversorgung, dadurch gekennzeichnet, daß in einer breit gewirkten oder geraschelten Bahn (100) aus saugfähigen, hydrophilen cellulosischen Fasergarnen (111, 112), wie z.B. Baumwolle, Zellwolle, Baumwolle/Zellwolle od.dgl. mindestens ein streifenförmiger Bahnabschnitt (120) aus hydrophoben Chemiefasergarnen aus synthetischen Polymeren mit einer hohen Oberflächenspannung, wie z.B. aus Polyamid, Polyester, Polyäthylen, Polypropylen, Polyacrylnitril, Polytetrafluoräthylen od.dgl., durch Vermaschung in einer Ebene eingearbeitet ist, wobei der streifenförmige Bahnabschnitt (120) als Mittelstreifen oder als Randstreifen in der Bahn (100) des textilen Flächengebildes (10) ausgebildet ist, das zu einer Kompresse (200) derart faltbar ist, daß der Bahnabschnitt (120) aus den hydrophoben Chemiefasern als hydrophobe Abdeckschicht (210) die Wundauflage und die hinter dem Bahnabschnitt (120) liegenden Lagen aus den cellulosischen Fasergarnen die Saugschicht oder die Saugschichten (220, 221, 222, 223) bildend sind.

2. Textiles Flächengebilde nach Anspruch 1, dadurch gekennzeichnet, daß die Dimension der verwendeten Garne zwischen 10 tex und 40 tex, vorzugsweise zwischen 14 tex und 20 tex liegen.

3. Textiles Flächengebilde nach Anspruch 1 und 2, dadurch gekennzeichnet, daß als Chemiefasergarne monofile und polyfile Filamentgarne und Stapelfasergarne verwendet werden, wobei die in dem streifenförmig ausgebildeten Bahnabschnitt (120) in Schußlegung geführten Fäden aus entsprechenden Materialien aufgebaut sind.

4. Textiles Flächengebilde nach Anspruch 1 bis 3, dadurch gekennzeichnet, daß die Breitgewirkebahn (100) in der Weise gefaltet ist, daß die hydrophobe Wundabdeckschicht (210) die Außenlage der Wundkompresse (200) und die dahinterliegenden Lagen aus hydrophilen Fäden die Saugschicht bzw. die Saugschichten (220, 221, 222, 223) bildend sind.

5. Textiles Flächengebilde nach Anspruch 1 bis 4, dadurch gekennzeichnet, daß zur Kennzeichnung der Wundauflagenseite die Chemiefasergarne aus synthetischen Polymeren mit einem indifferenten, physiologisch unbedenklichen Farbstoff, wie Pigmentfarbstoffe, geeignete Dispersions- oder Säurefarbstoffe od.dgl. eingefärbt sind.

6. Textiles Flächengebilde nach Anspruch 1 bis 5, dadurch gekennzeichnet, daß das die Wundkompresse (200) bildende textile Flächengebilde (10) antibakteriell oder medikamentös imprägniert ist, wobei als Imprägnierungsmittel quarternäre Ammoniumverbindungen, z.B. N-Cethylpyridiniumchlorid, Benzalkoniumchlorid u.dgl. sowie Stoffklassen mit desinfizierenden Eigenschaften oder aufgebrachte, mit Medikamenten, wie z.B. Perubalsam, Sulfonamide, Antibiotika, Anästhetika u.dgl. versetzte Vaseline verwendet werden.

7. Textiles Flächengebilde nach Anspruch 1 bis 6, dadurch gekennzeichnet, daß die Chemiefasergarne aus synthetischen Polymeren mit Silber, Aluminium oder anderen geeigneten Materialien oder Verbindungen oberflächlich beschichtet sind.

## Claims

1. A layered textile fabric (10) for the dressing of wounds, characterized in that, in a broad-width knitted or raschelknitted web (100) of absorbent, hydrophilic cellulosic spun yarns (111, 112), as e.g. cotton, spun rayon, cotton/spun rayon, etc., at least one strip-like web section (120) of hydrophobic manmade fibre yarn of synthetic polymers having a high surface tension, as e.g. polyamide, polyester, polyethylene, polypropylene, polyacrylnitrile, polytetrafluoroethylene, etc., is introduced by interconnection in one plane, wherein the strip-like web section (120) is formed as centre strip or marginal strip in the web (100) of the layered textile fabric (10), which can be folded so as to form a wound pad (200) in such a way that the web section (120) of the hydrophobic manmade fibres, as hydrophobic covering layer (210), forms the wound pad and the layers of cellulosic spun yarns located behind the web section form the absorbent layer or layers (220, 221, 222, 223).

2. A layered textile fabric according to Claim 1, characterized in that the dimensions of the yarns used range between 10 tex and 40 tex, preferably between 14 tex and 20 tex.

3. A layered textile fabric according to Claims 1 and 2, characterized in that, as manmade yarns, monofil and polyfil filament yarns and staple fibre yarns are used, wherein the threads guided in weft folding in the striplike configured web section (120) are composed of appropriate materials.

4. A layered textile fabric according to Claims 1 to 3, characterized in that the broad-width web of knitted fabric (100) is folded in such a way that the hydrophobic wound-covering layer (210) forms the outer layer of the wound pad (200) and the layers of hydrophylic yarns located behind the same form the absorbent layer or layers (220, 221, 222, 223).

5. A layered textile fabric according to Claims 1 to 4, characterized in that, for marking the wound pad side, the manmade fibre yarns of synthetic polymers are dyed with a neutral, physiologically safe dye, such as pigment dyestuffs, suitable disperse dyestuffs or acid dyestuffs, etc.

6. A layered textile fabric according to Claims 1 to 5, characterized in that the layered textile fabric (10) forming the wound pad (200) is antibacterially or medicinally impregnated, wherein, as impregnating agent, quarternary ammonia compounds, e.g. N-cethyl pyridium chloride, benzal conic chloride, etc., as well as material grades with desinfecting properties or applied vaseline compounded with medicines as e.g. Peru balsam, sulphonamides, antibiotics, anesthetics, etc., are used.

7. A layered textile fabric according to Claims 1 to 6, characterized in that the manmade fibre yarns of synthetic polymers are superficially coated with silver, aluminium or other suitable materials or compounds.

**Revendications**

1. Compresse en tissu (10) pour soigner les blessures, caractérisée en ce qu'au moins un tronçon de lé en forme de bande (120) en fils de fibres chimiques hydrophobes en polymères synthétiques à tension superficielle élevée, tels que par exemple en polyamide, polyester, polyéthylène, polypropylène, polyacrylonitrile, polytétrafluoréthylène ou équivalent, est incorporée par maillage dans un plan dans un lé tricoté de manière lâche ou en mailles Raschel lâches (100) en fils de fibres cellulosiques hydrophiles absorbantes (111, 112), comme par ex. le coton, la laine de cellulose, le mélange coton/laine de cellulose ou équivalent, le tronçon de lé en forme de bande (120) étant formé comme bande médiane ou comme bande marginale du lé (100) de compresse textile (10), qui est pliable en une compresse (200) de telle manière que le tronçon de lé (120) en fibres chimiques hydrophobes en tant que couche de recouvrement hydrophobe (210) forme la couche que l'on applique sur la blessure et que les couches derrière le tronçon de lé (120) en fils de fibres cellulosiques forment la couche absorbante ou les couches absorbantes (220, 221, 222, 223).

2. Compresse en tissu selon la revendication 1, caractérisée en ce que la dimension des fils utilisés est de l'ordre de 10 à 40 tex, de préférence de 14 à 20 tex.

3. Compresse en tissu selon les revendications 1 et 2, caractérisée en ce que l'on utilise des fils continus monofilaires et polyfilaires et des fils en fibres synthétiques à filer comme fils de fibres chimiques, les fils de trame du tronçon de lé (120) en forme de bande étant constitués par les matières correspondantes.

4. Compresse en tissu selon les revendications 1 à 3, caractérisée en ce que le lé à mailles lâches (100) est plié de telle manière que la couche hydrophobe de recouvrement de la blessure (210) forme la couche extérieure de la compresse (200) et que les couches en fils hydrophiles situées derrière celle-ci forment la ou les couches absorbantes (220, 221, 222, 223).

5. Compresse en tissu selon les revendications 1 à 4, caractérisée en ce que les fils de fibres chimiques en polymères synthétiques sont teints, pour caractériser la face à poser sur la blessure, avec une matière tinctoriale neutre, inoffensive sur le plan physiologique, comme des colorants à pigment, des colorants de dispersion ou des colorants acides appropriés ou équivalent.

6. Compresse en tissu selon les revendications 1 à 5, caractérisée en ce que la compresse en tissu (10) qui forme la compresse (100) est imprégnée de substance antibactérienne ou médicamenteuse, des composés quaternaires d'ammonium étant utilisés comme substances d'imprégnation, par ex. le chlorure de N-cetylpyridinium, le chlorure de benzalconium et équivalent, ainsi que des catégories de substances aux propriétés désinfectantes ou des vaselines appliquées, mélangées à des médicaments, tels que par ex. du baume du Pérou, des sulfamides, antibiotiques, anesthésiques ou équivalent.

7. Compresse en tissu selon les revendications 1 à 6, caractérisée en ce que les fils de fibres chimiques en polymères synthétiques sont enduits en surface d'argent, d'aluminium ou d'autres matières ou composés appropriés.

FIG.1

110    120    110

10

111    112    121    122    100

FIG.2

110    120    110    100

10(100)

A

110    120    110

C

120

110    110

D

110    120    110

B

200    110

E

200    120

F

*F I G. 3*